# EUROPEAN PATENT SPECIFICATION

(11) **EP 0 585 933 B1**
(45) Date of publication and mention of the grant of the patent: **21.12.2005**
(21) Application number: 93114091.7
(22) Date of filing: 02.09.1993
(51) Int. Cl.: G01N 27/30, A61N 1/04

(54) **Planar electrode**
Flache Elektrode
Electrode plane

(30) Priority: 04.09.1992 JP 23699892; 16.04.1993 JP 9029193
(43) Date of publication of application: 09.03.1994
(60) Divisional application: 03019671.1
(73) Proprietor: MATSUSHITA ELECTRIC INDUSTRIAL CO., LTD., Kadoma-shi, Osaka 571-8501 (JP)
(72) Inventor: Sugihara, Hirokazu, Katano-shi, Osaka 576 (JP); Taketani, Makoto, California 92714 (US); Mitsumata, Tadayasu, Hirakata-shi, Osaka 573 (JP)
(74) Representative: Vossius & Partner

(56) References cited:
- US-A- 4 971 853
- US-A- 5 388 577
- PATENT ABSTRACTS OF JAPAN vol. 016 no. 536 (P-1449) ,6 November 1992 & JP-A-04 204244 (MATSUSHITA ELECTRIC IND CO LTD) 24 July 1992, & DATABASE WPI Section Ch, Week 9236 Derwent Publications Ltd., London, GB; Class L03, AN 92-296180
- IEEE TRANSACTIONS ON BIOMEDICAL ENGINEERING, FEB. 1986, USA, vol. BME-33, no. 2, ISSN 0018-9294, pages 196-202, NOVAK J L ET AL 'Recording from the Aplysia abdominal ganglion with a planar microelectrode array'
- JOURNAL OF NEUROSCIENCE METHODS, FEB. 1980, NETHERLANDS, vol. 2, no. 1, ISSN 0165-0270, pages 19-31, PINE J 'Recording action potentials from cultured neurons with extracellular microcircuit electrodes'
- REVIEW OF SCIENTIFIC INSTRUMENTS, vol. 62, no. 9, September 1991 NEW YORK US, pages 2276-2280, PECKERAR M ET AL 'Passive microelectrode arrays for recording of neural signals: a simplified fabrication process'
- IEEE TRANSACTIONS ON BIOMEDICAL ENGINEERING, vol. BME-25, no. 6, November 1978 NEW YORK US, pages 494-500, MERCER H D ET AL 'Photolithographic fabrication and physiological performance of microelectrode arrays for neural stimulation'

## Description

This invention relates to a planar electrode which is used in the field of neurophysiology for electric measurement of biological activities, in particular, of the electric activities of nerve cells. This planar electrode comprises a large number of electrodes, and enables long-term culture of nerve cells on the electrodes, applies electric stimulation to nerve cells through electrodes during culture period, and is able to record electrical activities of nerve cells using the electrodes.

Recently, medical investigations on nerve cells and investigations of the possibility of using nerve cells as electronic elements have been actively pursued.

The interior of a nerve cell is separated from the exterior by the cell membrane, through which substances are delivered to and from the cell. The cell membrane exhibits different permeability to various substances. This property is called selective permeability.

The cell membrane of nerve cells exhibits selective permeability also to various types of ions. Moreover, this selectivity varies in accordance with the cell condition. Inside the nerve cell in the resting state, the concentration of Na⁺ is low and that of K⁺ is high. Conversely, outside the cell, the concentration of Na⁺ is high and that of K⁺ is low. As a result, an ion concentration gradient is produced between the inside and the outside of the cell, and a potential difference (membrane potential) is developed. This membrane potential is negative inside the cell with respect to the outside of the cell and is generally around -70 mV for vertebrates. This membrane potential is called the resting membrane potential.

In the event that stimulation (generally electrical stimulation) is given to nerve cells and the membrane potential is changed from resting to positive (depolarization), if the magnitude of depolarization is below a certain threshold value, the selective permeability of the cell membrane to ions does not change and the action potential is not generated. When depolarization exceeds the threshold value, the selective permeability of the cell membrane to ions changes and the action potential is generated. As the action potential is generated, the ion concentration inside and outside the cell membrane varies. Measuring this potential change accompanied by the ion concentration change near the nerve cells (that is, the ion current) with electrodes enables the detection and investigation of nerve activities.

Conventionally, in order to measure the electrical activities of nerve cells, it is common practice to use a recording electrode and a stimulating electrode comprising glass, metal, or other electrodes, insert them in or between cells, and measure the electrical activities of nerve cells with the recording electrode when a stimulating current (or voltage) is applied from the stimulating electrode.

In addition to this, there are many modified methods such as the so-called whole cell patch clamp method, in which a cell is absorbed within a glass electrode formed into a dropping pipet with an about 1-µm-diameter tip end designed to break a part of the cell membrane, the inside of the cell body is refluxed with the liquid in the glass electrode, and electrical signals are emitted from this glass electrode to observe electric characteristics of the cell.

In the conventional technique and its modified methods, electrodes such as glass electrodes, which have to be larger than the cells themselves, must be used. As a result, primarily due to restrictions of space and operating accuracy, multi-point simultaneous measurements in which two or more recording electrodes are inserted simultaneously in one sample to record electrical activities of the nerve cells are extremely difficult.

In order to investigate the operation of the whole nerve circuit network, it is necessary to record many nerve cell activities simultaneously, and as the number of measuring points increases, the degree of difficulty increases, creating the problem that it is difficult to observe throughout a large number of cells.

In addition, because glass, metal, or other electrodes must be pierced into or between cells, there is another problem that the damage to the cell is serious and measurement over a long time such as extending for more than a few hours is difficult to carry out.

Because in the whole cell patch clamp method, the cell inside is refluxed with the liquid contained in the glass electrode, damage to the cell is even greater, restricting the measurement time to about 1 hour.

JP-A-04204244 and the English abstracts in PATENT ABSTRACTS OF JAPAN vol. 016 no. 536 (P-1449); DATABASE WPI Section Ch., Week 9236 Derwent Publications Ltd., London, GB; Class- L03, AN 92-296180 relates to an integrated, composite electrode for measuring electrical activity of nerve cells. The purpose of this electrode is to make simple multi-point concurrency measurement and to make the observation of the signal transfer extending among many cells. Complex electrodes are incorporated in one and are provided with a wiring element wherein lead wires from the plural electrodes are radially arranged. The distances between the nearest electrodes are equal to each other. Furthermore an insulating layer is provided having holes on the electrode.

Thus, the whole device comprises an insulating baseplate, a patterned conductive layer and a patterned insulation layer. The conductive layer is patterned to create electrode paths and leads. The insulation layer is made of polyimide and comprises one hole above each electrode, thus the top insulation layer is patterned to expose only a part of the conductive layer. The exposed area of the conductive layer acts as a electrode which can contact with neurons and solutions. Thus, the effective electrode is the part which is not covered by the insulation layer and the size thereof corresponds to the size of the hole. Only this part of the electrode can contact with neutrons and solution.

Therefore, the electrodes disclosed have a diameter of 15 µm, but the effective size of the electrode corresponds to the size of the hole which has a diameter of 10 µm in the described embodiment. Thus, the size of the effective electrode is 78.5 µm² (=5 x 5 x 3,14). It is mentioned that the size of the hole may range from 5 to 20 µm TRANSACTIONS ON BIOMEDICAL ENGINEERING; Feb: 1986, USA, vol. BME-33, no.2, ISSN 0018-9294, pages 196-202, NOVAK J L ET AL: ,Recording from the Aplysia abdominal gangolin with a planar microelectrode array relates to a passive multi-microelectrode array which has been fabricated and used to record neural event from the abdominal ganglion of the marine mollusk, *Aplysia californica*. The array consists of a pattern of gold conductor lines on a glass substrate which is insulated with a polyimide. The 32 electrodes are 25 µm in diameter and are arranged in a 4 x 8 matrix on 200 µm centers. Above each electrode a 10 µm diameter hole has been provided. On top of the array a well for culture medium is provided.

JOURNAL OF NEUROSCIENCE METHODS; Feb. 1980, Netherlands, vol. 2, no. 1, ISSN 0165-0270, pages 19-31, Pine J: 'Recording action potentials from cultured neurons with extra-cellular microcircuit electrodes' refers to recording action potentials from cultured neurons with extra cellular microcircuit electrodes. Dissociated cell cultures of neurons from neonatal rat superior cervical ganglia have been grown in specially prepared dishes, the bottoms of which consist of glass coverslips on which thin-film microcircuits have been deposited. The microcircuit provides 32 microelectrodes per dish, each approximately 8 x 10 µm in area. Extracellular recordings of action potentials from individual neurons have been made, with good signal-to noise ratios, for cells within 40 µm of the electrode centers. However subthreshold synaptie potentials as an example have not been seen.

IEEE Transactions on Biomedical Engineering; Nov. 1978, Vol. BME25; no. 6, pages 494-500 describes a microelectrode array therein comprising stimulation electrodes having an effective electrode area of 75 µm in diameter. This corresponds to an area of 4,418 µm². However, this device is adopted for in vivo use.

Accordingly, a feature of the invention is to provide a planar electrode which solves the problems at the prior art and enables easy multi-point simultaneous stimulation and measurement of nerve cells as well as signal transmission and observation throughout many cells for more than just a few hours.

These problems are solved by the features of claim 1.

The invention features a planar electrode comprising an insulating substrate, a multiplicity of electrodes placed thereon with adjacent electrodes being equidistantly spaced, a wiring section in which lead wires are installed substantially radially from the electrodes, and an insulating layer covering the lead wires. The electrodes each have an area 2500 µm². A well is formed on top of the array.

It is preferable in the invention that the shortest electrode-to-electrode distance is from 10 to 1000 µm.

It is preferable in the invention that the insulating layers covering the lead wires have holes on each electrode and are provided nearly all over the surface of the insulating substrate except in the vicinity of the contacts of the lead wires with an external circuit.

It is preferable in the invention that the surface resistance of the electrode is 10 Ω/cm² or lower. For this reason, it is preferable to cover the surface of the electrode with either platinum, platinum black, or gold.

It is preferable in the invention that the center of a multiplicity of electrodes is located on each intersection of an 8×8 lattice.

It is desirable for the insulating substrate to be made of a transparent material, so that the nerve cell cultured on the insulating substrate can be easily observed. For similar reasons, it is desirable for the electrodes, the lead wires and the insulating layer to be made of a transparent material.

It is further preferable in the invention that the electrode and the lead wire each are made from indium tin oxide (ITO), tin oxide. Cr, Au, Cu. Ni, or Al.

In particular, the insulating layer is preferably made from either polyimide (PI) resin, negative photosensitive polyimide (NPI) resin, epoxy resin, acrylate resin, polyester resin, or polyamide resin.

The planar electrode of the invention enables detection of the transmission of signals between adjoining cell bodies in providing signals to nerve cells cultured on the planar electrode of the invention and measuring the signal between cells at the same time. This is because one cell body is arranged on the electrode, and it can be arranged with a high degree of probability that the cell body mediating the cell protrusions (e.g., dendrites and axons on nerve cells) will be located on adjoining electrodes by adjusting the shortest electrode-to-electrode distance to be nearly equal to the length of a nerve cell to be measured (that is, cell body, dendrites, axon) and equally spacing the electrodes.

Furthermore, arranging the lead wires extending from the electrodes substantially radially reduces the capacitive component (capacitance) between lead wires from the capacitance when they are arranged in parallel. The collapse of pulse signal waveform. Of electrical signals, is also reduced, and the time constant of the circuit becomes small, improving the response to quick pulse signals and therefore improving the follow-up to the component with fast nerve cell activities.

In addition, adjusting the electrode area enables application of electric stimulation to a cell over a long time exceeding few days as well as measurement of electric activities of the cell. In particular, in the lower end of the electrode area range, the planar electrode of the invention is suited for recording subtle cell activities, while in the higher end of the electrode area range, the invention is suited for applying electric stimulation to cells over a long time. Adjusting the electrode area enables designing a planar electrode suited for a variety of applications.

In addition, in the planar electrode of the invention, bringing the shortest electrode-to-electrode distance to the desirable condition of 10-1000 µm results in a high possibility of locating the cell bodies on adjoining electrodes and connecting them via their axons, achieving an electrode-to-electrode distance convenient for investigating information transmission between nerve cells.

In the planar electrode of the invention, the insulating layer, in which the insulating layers covering lead wires have holes over each electrode and are installed on nearly the whole surface of the insulating substrate except the vicinity of the section where the lead wire comes in contact with the external circuit, allows easy formation of the required insulating layer by applying the insulating material comprising photo-sensitive resin to nearly the whole surface and removing the insulating layer on each electrode by a photoetching method and opening holes to expose electrodes, thereby achieving easy production and minimizing the probability of insulation failure, which is very desirable.

Designing the electrodes and lead wires to be formed with transparent material enables easy observation of nerve cells cultured on the planar electrode, which is also very desirable.

Determining the surface resistance of the electrode section to achieve the desired range of 10 Ω/cm² or less substantially prevents polarization on the stimulating electrode surface of the nerve cell from occurring when a stimulating current is applied for a long time to nerve cells at a certain electrode and recording electrical activities (potential change) of the nerve cells corresponding to the stimulating current at other electrodes, thereby minimizing the effects (that is artifacts) of stimulating current on potential recorded waveform. In particular, because even after a stimulating current is applied over a long time, with the invention artifacts are small and the mode is free from change, so electrical activities of nerve cells before and after long stimulation can be compared.

In addition, when the electrode surface is covered with either platinum, platinum black, or gold to bring down the surface resistance of the electrode to 10 Ω/cm² or less, the platinum, platinum black, or gold comes into direct contact with nerve cells; because these metals are known to cause low cytotoxicity, they are suitable to achieve the objectives of the invention.

Furthermore, in the planar electrode of the invention, locating the center of a multiplicity of electrodes at each intersection of 8 × 8 lattices secures the maximum number of electrodes which enables installation of lead wires substantially radially from the electrodes of the invention, which is very desirable.

This invention provides a planar electrode with excellent response capable of culturing nerve cells and achieving simultaneous multi-point measurement of electrical activities of nerve cells and long-term observation of signal transmission over many cells for more than several hours, which conventionally has been an impossible or very difficult result to achieve.

The required insulating layer pattern is easily formed by the photoetching method and provides a planar electrode which is easy to produce and has small probability of insulation failure. This method can readily produce an electrode in which the insulating layers on lead wires have holes over each electrode and are installed on nearly the whole surface of the insulating substrate except in the vicinity of the sections where the lead wires come in contact with the external circuit, as compared with a planar electrode in which an insulating layer is provided merely on the lead wires. This is achieved, for example, by applying an insulating material comprising photo-sensitive resin on nearly the whole surface and removing the insulating layer on each electrode to make a hole for exposing the electrode.

Because each electrode has small surface resistance and is coated with a substance of low cytotoxicity, when suitable electrodes are used to apply stimulating current and the potential change is recorded with other suitable electrodes, the invention provides a planar electrode with less polarization of electrodes and which provides stable recording even after stimulation is applied over a long time.

In addition, the preferred embodiment of the invention in which a multiplicity of electrodes each are located at intersecting points of 8 × 8 lattices provides a planar electrode with maximum number of electrodes which allows installation of lead wires nearly radially from the electrodes.
FIG. 1 is a plan view of an embodiment of the invention prior to the application of an insulating layer on the planar electrode of the invention showing electrodes and lead wires formed on an insulating substrate
FIG. 2 is a partially notched view of the plan view of Fig. 1 having the insulating layer of an embodiment of the invention applied to a planar electrode;
FIG. 3 is a fragmentary cross sectional view of an embodiment of a planar electrode of the invention;
FIG. 4 shows a changing potential waveform recorded with other suitable electrodes before applying stimulating current over a long time using suitable electrodes in an embodiment of the planar electrode of the invention;
FIG. 5 shows the changing potential waveform recorded with other suitable electrodes after applying stimulating current over a long time using suitable electrodes in an embodiment of the planar electrode of the invention;
FIG. 6 shows the changing potential waveform recorded with other suitable electrodes before applying stimulating current over a long time with suitable electrodes, using a planar electrode which differs from the planar electrode of the invention only in that the electrode surface is not coated with gold;
FIG. 7 shows another kind of the changing potential waveform recorded with electrode after applying stimulating current over a long time with suitable electrodes, using a planar electrode which differs from the planar electrode of the invention only in that the electrode surface is coated with gold.

As the insulating substrate material used for the invention, a transparent substrate is desirable because microscopic observation is required after the cell culture as described above. Examples include glasses such as quartz glass, lead glass or borosilicate glass, or inorganic substances such as quartz, or organic substance with transparency such as polymethyl methacrylate or its copolymers, polystyrene, polyvinyl chloride, polyester, polypropylene, urea resin, and melamine resin. Considering mechanical strength in combination with transparency, though, inorganic substances are desirable.

As the electrode materials used for the invention, examples include indium tin oxide (ITO), tin oxide, Cr, Au, Cu, Ni and Al. In particular, the use of ITO or tin oxide produces a slightly yellowish transparent electrode which provides good visibility of nerve cells under the microscope and is advantageous in experimental operation, but ITO is particularly desirable for its good conductivity.

The same materials can be applied to lead wires, and ITO is again desirable for similar reasons to those mentioned for electrode materials.

It is not a particular restriction of the invention, but in general the thickness of the electrodes and lead wires should be about 50-500 nm and, in general, these materials are evaporated on the insulating substrate and formed in a desired pattern by etching using a photoresist.

As the insulating layer material used to insulate the lead wires used for the invention, examples include polyimide (PI) resin, epoxy resin, acrylate resin, polyester resin, polyamide resin, and other transparent resins.

These types of resin are applied on lead wires by conventional techniques to form an insulating layer. When the insulating material is a photosensitive resin which has photochemical polymerization properties, etc., it is desirable because patterns can be formed to provide holes on the insulating layer portion on the electrodes to expose the electrodes as described above.

In particular, when insulating material is PI and the cell to be cultured is a nerve cell, satisfactory growth takes place and therefore, it is very desirable. In addition, among types of PI, negative photosensitive polyimide (NPI) is most desirable because holes can be formed on the electrodes using a photo etching process after the negative photosensitive polyimide is applied over nearly the whole surface, in a manner similar to that in forming patterns of the wiring section.

The thickness of the insulating layer may be such that can impart insulating capability. This is not particularly limiting but in general, a thickness of 0.1-10 µm, specifically, one of 1-5 µm is desirable.

The planar electrode of the invention directly cultures cells and measures and records electrical activities of the cells. Depending on culture conditions or the type of cells, the size of cell body or the length of cell protrusion such as dendrites or axons may vary but 10-1000 µm is desirable for the electrode-to-electrode distance of the closest planar electrodes. When the electrode-to-electrode distance is less than 10 µm, the electrodes are so close to one another that the probability for the cell bodies to adjoin via cell protrusions decreases. Furthermore, wiring of lead wires become difficult. When the electrode-to-electrode distance exceeds 1000 µm, lead wires can be easily wired but as it is rare for the cell protrusions of cultured nerve cell to elongate as far as about 1000 µm, the probability of the cell body to be located on the electrode decreases. Even under general conditions, about 200-300 µm is desirable for the electrode-to-electrode distance because the length of cell protrusions of a cultured cell is about 200-300 µm on average for central nervous system cells of mammals.

With respect to the electrode area, in order to avoid electrode breakage when electric stimulation is applied to the cell over a long time, it is necessary to reduce resistance at the interface with the culture medium, requiring a size exceeding a certain level. However, as the electrode area increases and the resistance at the interface with the culture medium reduces, the electric activity of the cell to be measured decreases and the signal to noise (S/N) ratio decreases. That is, if the current value I is constant, it follows from I = V/R that the potential V to be measured reduces with decreasing resistance R. That is, the electrical activities of the cell to be measured decrease and the S/N ratio lowers. Consequently, the electrode area must carefully be adjusted.

In order to bring the surface resistance of the electrode section down to 10 Ω/cm² or less, the ITO top surface is coated with metal. Examples of coating material include Ag, Al, Bi, Au, Cu, Cr, Pt and Co, but with low toxicity to nerve cells taken into account, the use of Au or Pt is desirable. The coating thickness is not, in particular, limited, but is about 50 nm and, in general, these materials are evaporated on the insulating substrate and are formed into desired patterns by etching using a photoresist.

By dripping a platinum salt solution on the electrode and electrolyzing it to deposit platinum black on the electrode, it is possible to coat the electrodes.

In addition, according to a preferred embodiment of the invention described previously, holes in the insulating layer of the planar electrode are formed to expose electrodes not only to give electrical stimulation to the cell body cultured on the planar electrode but also to detect electrical activities from adjoining cell bodies which are located at the central portion of the electrode.

Arranging lead wires stretched from the electrode nearly radially eliminates the capacitance between lead wires, reduces the time constant, and improves the measuring accuracy.

The configuration in which the electrode center portion of the planar electrode of the invention is located at each intersection of lattices of 8×8 or smaller enables the lead wire to be radially installed, and from the viewpoint of particularly forming as many electrodes as possible and providing and recording multi-point stimulation simultaneously, it is desirable to install electrodes at each intersection of the 8×8 lattices.

Now referring the following specific embodiments, the planar electrode of the invention will be described in further detail.

### (Embodiment 1)

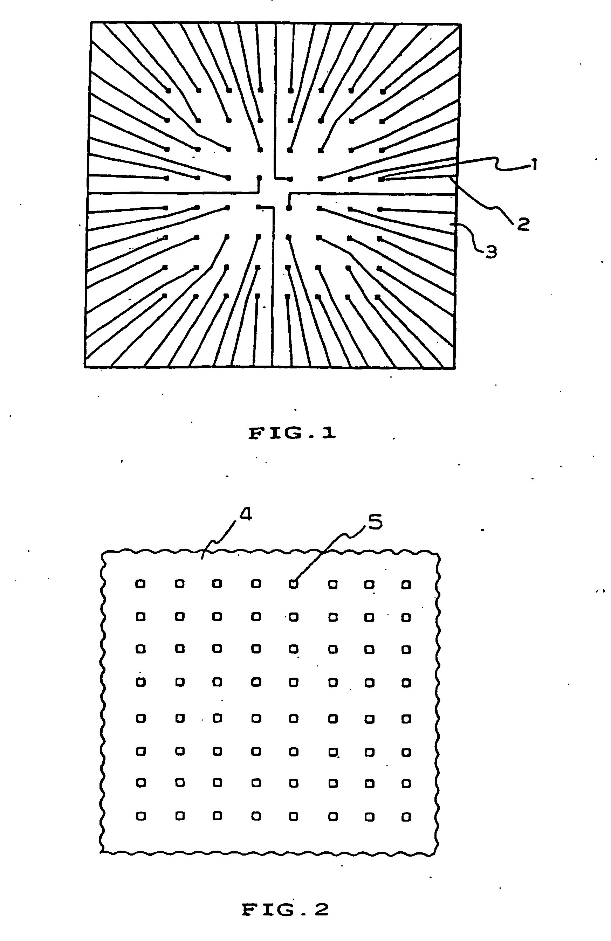

First, fabrication of a planar electrode wiring section is described. As the insulating substrate 3 of the planar electrode in Figs. 1 and 3, 50 × 50 × 1 mm hard glass ("IWAKI CODE 7740 GlASS" - Iwaki Glass Co., Ltd.) was used; this is a transparent insulating material with high mechanical strength.

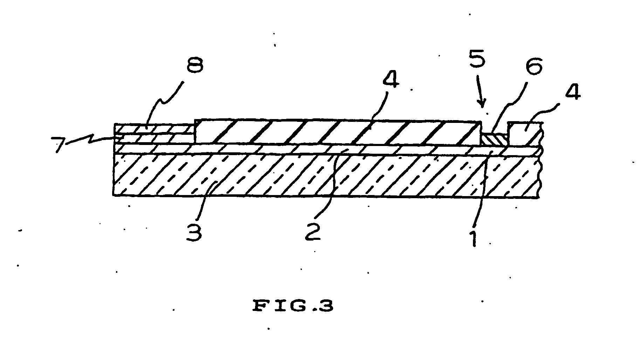

Referring to Fig. 3, for the material of electrode 1 and lead wire 2, ITO was used, and on the whole surface of the insulating substrate 3 of the hard glass, ITO was evaporated to form a layer about 100 nm thick, which was followed by rinsing.

Then, the substrate was exposed to light through a photoresist so that the central portion of each electrode 1 was located on each intersection of 8×8 lattices (position 5 as shown in Fig. 2), the center-to-center distances of nearest electrodes of each electrode were equal, and lead wire 2 formed the pattern of electrode 1 and lead wire 2 in which lead wire 2 was stretched radially. It was then etched with ITO in a solution which was made up using demineralized water, hydrochloric acid, and nitric acid in a volume ratio of 50:50:1, and the photoresist was removed. The wiring portion with electrode 1 being 60 µm in diameter, lead wire 2 being 30 µm wide, and a center-to-center distance of electrodes of 300 µm was thus formed.

Then, for insulating layer 4, negative photo-sensitive polyimide (hereinafter called "NIP") was spin-coated so that a film 1 µm thick was formed after drying, and an insulating layer pattern was exposure-formed so that a 50 µm square hole 5 was produced at the center of each electrode of the wiring section as shown in Fig. 2. Furthermore, to the exposed portion of each electrode (that is, the inside of the 50 µm square), gold 6 was evaporated in a film thickness of 50 nm.

The contact with the external circuit of the section near the end opposite to electrode 1 of lead wire 2 was coated with gold 7 and nickel 8 to improve durability.

In this embodiment, ITO was used for electrode 1 and lead wire 2, NPI for the insulating layer, and gold for the electrode surface coating material, but it has already been stated that the material used shall not be limited to these.

The process of producing the planar electrode of the invention is not limited to the method described in this embodiment.

### (Embodiment 2)

Next, the culture of nerve cells on the planar electrode is described.

On the planar electrode formed in Embodiment 1, cerebral visual cortex cells of rats were cultured as the nerve cells.

Now, the culture method will be discussed in detail.
(a) Brains of fetuses of SD rats at 14-18 days of pregnancy were removed and immersed in iced Hanks' Balanced Salt Solution (hereinafter called "HBSS").
(b) From the brains in the iced HBSS, visual cortices were cut out and transferred to Eagle's minimum essential medium (hereinafter called "MEM") liquid.
(c) In the MEM liquid, the visual cortices were cut into as small pieces as possible, 0.2 mm square at maximum.
(d) The visual cortices cut into small pieces were placed in centrifugal tubes (test tubes for centrifugal separation), and after washing with HBSS free from calcium and magnesium (hereinafter called "CMF-HBSS") three times, they were dispersed in a suitable volume of the same liquid.
(e) In the centrifugal tubes of Step (d), a CMF-HBSS solution of trypsin (0.25 wt%) was added to double the total volume. With gentle stirring, enzymatic processes were allowed to take place while the solution was incubated at 37 °C for 15 to 20 minutes.
(f) DMEM/F-12 mixture medium in which Dulbecco modified Eagle's medium (DMEM) and HamF-12 medium were mixed in a volume ratio of 1:1 was added to the centrifugal tube subjected to Step (e) to further double the total volume. With a Pasteur pipette with a reduced diameter produced by fire-polishing the tip end with a burner, gently repeating pipetting (about 20 times at maximum), the cells were unravelled.
(g) Centrifugation was carried out for about 5 minutes at 9806.65 m/sec² (that is, 1000 g). Upon completion of centrifugation the supernatant was discarded and the precipitate was suspended in DMEM/F-12 mixture medium containing FCS 5%.
(h) Step (g) was repeated two more times (a total of 3 times).
(i) The precipitate finally obtained was suspended in the DMEM/F-12 mixture medium containing 5% FCS and using an erythrocytometer, the cell concentration in the suspension liquid was measured. Using the similar medium, the cell concentration was adjusted to be 1 × 10⁶ to 5 × 10⁶ cells/mL.
(j) In a well for cell culture formed by affixing a plastic cylinder 25 mm in diameter and 6 mm high to the planar electrode with the planar electrode center aligned with the plastic cylinder center, 500 µL of the DMEM/F-12 mixture medium containing 5% FCS was added in advance and heated in a CO₂ incubator (air content: 95 vol%; CO₂ content: 5 vol%; relative humidity: 97%; Temperature: 37°C).
(k) In the well of Step (j), 100 µL of the suspension liquid with the cell concentration adjusted was gently added and again let stand in the CO₂ incubator.
(l) Three days after the performance of Step (k), one half the medium was replaced with a new one. For the replaced medium, the DMEM/F-12 mixture medium not containing FCS was used.
(m) Thereafter, half of the medium was replaced in the similar manner every 4 to 5 days.

Over the series of these operations, nerve cells of cerebral visual cortices of rats were cultured on the planar electrodes.

The cells grew successfully even on the insulating layer (NPI) and even on the electrodes with gold evaporated on them. Consequently, the use of the electrodes suitably located for the stimulation electrodes or recording electrodes enabled the simultaneous multi-point measurement of the electrical activities of nerve cells.

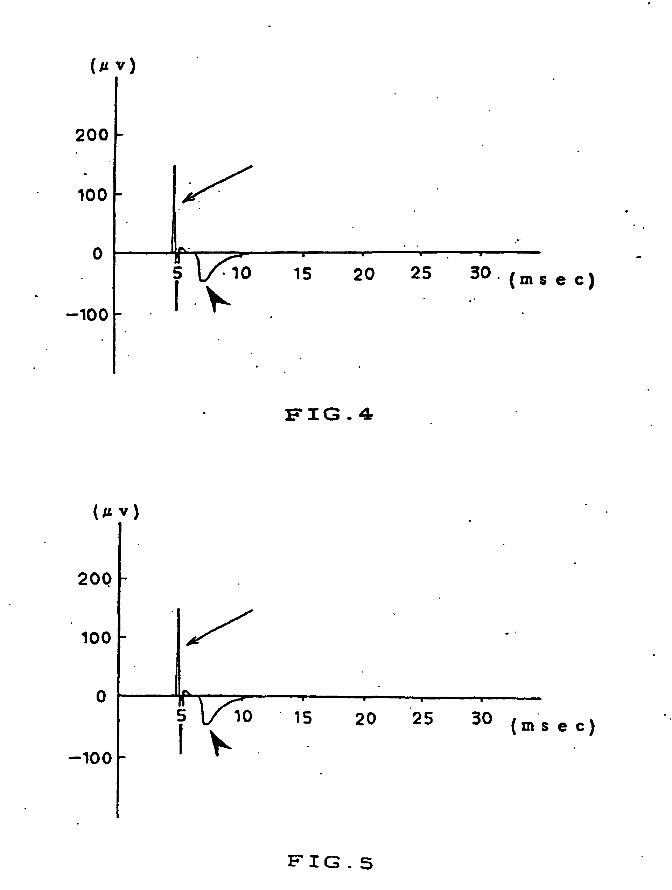

Figs. 4 and 5 show examples of electrical responses (potential changes) of nerve cells at the electrodes located at suitable places recorded before and after constant current stimulation of 100 µA was provided over one week at a frequency of 1 Hz via the electrodes suitably located in the planar electrode of the invention. Fig. 4 shows the electrical responses of the nerve cells before stimulation, while Fig. 5 shows the electrical responses of the nerve cells after stimulation.

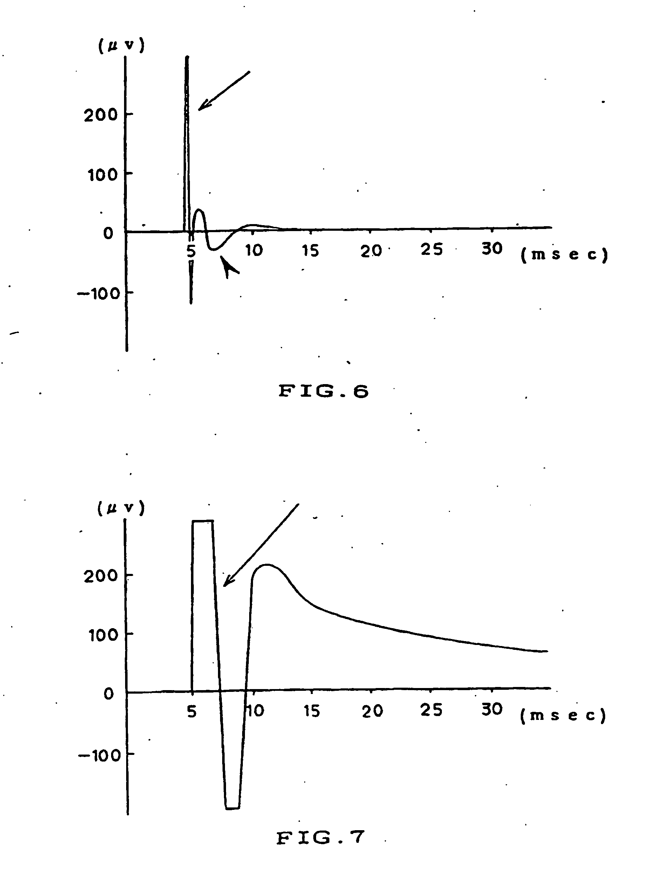

In addition, Figs. 6 and 7 show examples of electrical responses of nerve cells recorded before and after long-term stimulation was applied under the same conditions as above, using a planar electrode whose surface is not coated with gold. Fig. 6 shows the records of electrical response of nerve cells before stimulation, while Fig. 7 shows the records of electrical response of nerve cells after stimulation.

In Figs. 4 through 7, the arrow marks show the artifacts generated as a result of application of stimulation current and the arrow head shows a potential change generated by electrical activities of nerve cells.

As is clear from Fig. 6, when the planar electrode whose surface is not coated with gold is used, generation of artifacts is great, while when the planar electrode of one embodiment of the invention shown in Fig. 4 is used, generation of artifacts is suppressed.

As is clear from Fig. 7, when the planar electrode surface is not coated with gold, generation of artifacts is greater after than before stimulation; the electrical activities of the nerve cells are hidden by the artifacts and measurement is disabled. In contrast, when the planar electrode of one embodiment of the invention as shown in Fig. 5 is used, same as in the case shown in Fig. 4, generation of artifacts is suppressed and the electrical activities of the nerve cells are successfully recorded.

There are many other methods than those described above for the culture of nerve cells on the planar electrode of the invention.

The invention may be embodied in other specific forms. The disclosed embodiments are to be considered in all respects as illustrative and not restrictive, the scope of the invention being indicated by the appended claims rather than by the foregoing description and all changes which come within the meaning and range of equivalency of the claims are intended to be embraced therein.

## Claims

1. A planar electrode an array having comprising:
a) an insulating substrate (3) comprising one or a plurality of insulating layers;
b) one or a plurality of patterned conductive layers (1,2) on said substrate (3) which creates a plurality of nearly radially installed narrow conductive strips;
c) a patterned insulation layer (4) on said conductive layers (1,2); where a combination of said narrow conductive strips and said patterned insulation layer (4) creates one or a plurality of de-insulated conductive parts (5) in each said narrow conductive strip as terminals and one de-insulated conductive part (5) in each said narrow conductive strip as an electrodes having an area of 2.5x10³µm² and a well for cell culture on the top of the planar electrocle array.

2. The planar electrode according to claim 1 wherein the distance between adjacent electrodes is from 10 to 1000 µm.

3. The planar electrode according to claim 1 or 2, wherein the insulating layer covering the lead wires has holes over each electrode and is provided over substantially the entire surface of said insulating substrate except in the vicinity of contacts between an external circuit and the lead wires.

4. The planar electrode according to claim 1 or 2, wherein the insulating substrate comprises a transparent material.

5. The planar electrode according to claim 1 or 2, wherein the electrodes and the lead wires are made of a transparent material.

6. The planar electrode according to claim 1 or 2, wherein the electrodes and the lead wires each are made from indium tin oxide, tin oxide, Cr, Au, Cu, Ni, or Al.

7. The planar electrode according to claim 1 or 2, wherein said insulating layer comprises a transparent material.

8. The planar electrode according to claim 1 or 2, wherein said insulating layer is made from polyimide resin, negative photosensitive polyimide resin, epoxy resin, acrylate resin, polyester resin, or polyamide resin.

9. The planar electrode according to claim 1 or 2, wherein the surface resistance of said electrode is 10 Ω/cm² or lower.

10. The planar electrode according to claim 1 or 2, wherein surfaces of said electrodes are covered with platinum, platinum black, or gold.

11. The planar electrode according to claim 1 or 2, wherein centers of the electrodes are located at each intersection of an 8×8 lattice.

12. The planar electrode according to claim 3, wherein one or more of the insulating substrate, the electrodes, the lead wires and the insulating layer is made of a transparent material.

13. The planar electrode according to claim 12, wherein one or both of the electrodes and the lead wires is made from indium tin oxide, tin oxide, Cr, Au, Cu, Ni, or Al.

14. The planar electrode according to claim 12, wherein the insulating layer is made from polyimide resin, negative photosensitive polyimide resin, epoxy resin, acrylate resin, polyester resin or polyamide resin.

15. The planar electrode according to claim 3, wherein surfaces of said electrodes are covered with platinum, platinum black or gold.

16. The planar electrode according to claim 3, wherein centers of the electrodes are located at each intersection of an 8×8 lattice.

17. Use of a planar electrode array, according to any of the forgoing claims for capturing and/or producing a signal waveform of electrical activity in biological tissue and cell.

18. Use according to claim 17 wherein said biological tissue and cells are taken from a nervous system.

19. Use according to claims 17 or 18 wherein the electrical activity before and after long stimulation preferably of more than one week is compared.

20. Use according to any of claims 17 to 19 wherein a stimulating current is applied to biological tissue or cells at a certain electrode and wherein electrical activities of the biological tissue or cell corresponding to the stimulating current are recorded at other electrodes.

## Patentansprüche

1. Flache Elektrode mit einer Anordnung, die aufweist:
a) ein isolierendes Substrat (3), das eine oder mehrere isolierende Schichten aufweist;
b) eine oder mehrere mit Muster versehene leitende Schichten (1,2) auf dem Substrat (3), was mehrere fast radial angebrachte schmale leitenden Streifen erzeugt;
c) eine mit Muster versehene Isolationsschicht (4) auf den leitenden Schichten (1,2); wobei eine Kombination aus den schmalen leitenden Streifen und der mit Muster versehenen Isolationsschicht (4) einen oder mehrere nicht-isolierte leitende Abschnitte (5) in jedem schmalen leitenden Streifen als Anschlüsse und einen nicht-isolierten leitenden Abschnitt (5) in jedem schmalen leitenden Streifen als Elektrode mit einer Fläche von 2,5x10³ µm² erzeugt; und
eine Kammer für eine Zellkultur auf der Oberseite der flachen Elektrodenanordnung.

2. Flache Elektrode nach Anspruch 1, wobei der Abstand zwischen benachbarten Elektroden zwischen 10 und 1000 µm ist.

3. Flache Elektrode nach Anspruch 1 oder 2, wobei die isolierende Schicht, die die Leitungsdrähte bedeckt, über jeder Elektrode Löcher hat und im wesentlichen über die gesamte Oberfläche des isolierenden Substrats hinweg bereitgestellt ist, mit Ausnahme in der Nähe der Kontakte zwischen einer externen Schaltung und den Leitungsdrähten.

4. Flache Elektrode nach Anspruch 1 oder 2, wobei das isolierende Substrat ein transparentes Material aufweist.

5. Flache Elektrode nach Anspruch 1 oder 2, wobei die Elektroden und die Leitungsdrähte aus einem transparenten Material bestehen.

6. Flache Elektrode nach Anspruch 1 oder 2, wobei sowohl die Elektroden als auch die Leitungsdrähte aus Indiumzinnoxid, Zinnoxid, Cr, Au, Cu, Ni oder Al hergestellt sind.

7. Flache Elektrode nach Anspruch 1 oder 2, wobei die isolierende Schicht ein transparentes Material aufweist.

8. Flache Elektrode nach Anspruch 1 oder 2, wobei die isolierende Schicht aus Polyimidharz, negativem lichtempfindlichem Polyimidharz, Epoxyharz, Acrylatharz, Polyesterharz oder Polyamidharz hergestellt ist.

9. Flache Elektrode nach Anspruch 1 oder 2, wobei der Oberflächenwiderstand der Elektrode 10 Ω/cm² oder weniger ist.

10. Flache Elektrode nach Anspruch 1 oder 2, wobei die Oberflächen der Elektroden mit Platin, Platinschwarz oder Gold bedeckt sind.

11. Flache Elektrode nach Anspruch 1 oder 2, wobei der Mittelpunkt einer Elektrode auf einem Schnittpunkt eines 8x8-Gitters angeordnet ist.

12. Flache Elektrode nach Anspruch 3, wobei das isolierende Substrat und/oder die Elektroden und/oder die Leitungsdrähte und/oder die isolierende Schicht aus einem transparenten Material hergestellt ist.

13. Flache Elektrode nach Anspruch 12, wobei die Elektroden und/oder die Leitungsdrähte aus Indiumzinnoxid, Zinnoxid, Cr, Au, Cu, Ni oder Al hergestellt sind.

14. Flache Elektrode nach Anspruch 12, wobei die isolierende Schicht aus Polyimidharz, negativem lichtempfindlichem Polyimidharz, Epoxyharz, Acrylatharz, Polyesterharz oder Polyamidharz hergestellt ist.

15. Flache Elektrode nach Anspruch 3, wobei die Oberflächen der Elektroden mit Platin, Platinschwarz oder Gold überzogen sind.

16. Flache Elektrode nach Anspruch 3, wobei der Mittelpunkt jeder Elektrode auf einem Schnittpunkt eines 8x8-Gitters angeordnet ist.

17. Verwendung einer flachen Elektrodenanordnung nach einem der vorstehenden Ansprüche, zum Erfassen und/oder Erzeugen einer Signalwellenform einer elektrischen Aktivität in einem biologischen Gewebe oder einer Zelle.

18. Verwendung nach Anspruch 17, wobei das biologische Gewebe und die Zellen dem Nervensystem entnommen sind.

19. Verwendung nach Anspruch 17 oder 18, wobei die elektrische Aktivität vor und nach einer langen Stimulation vorzugsweise von mehr als einer Woche verglichen wird.

20. Verwendung nach einem der Ansprüche 17 bis 19, wobei an einer bestimmten Elektrode dem biologischen Gewebe oder den Zellen ein Stimulationsstrom zugeführt wird und wobei elektrische Aktivitäten des biologischen Gewebes oder der Zellen, die dem Stimulationsstrom entsprechen, an anderen Elektroden aufgezeichnet werden.

## Revendications

1. Electrode plane comprenant un réseau comportant
a) un substrat isolant (3) comprenant une ou une pluralité de couches isolantes ;
b) une ou une pluralité de couches conductrices configurées (1, 2) sur ledit substrat (3), qui créent une pluralité d'étroites bandes conductrices à disposition presque radiale ;
c) une couche isolante (4) configurée sur lesdites couches conductrices (1, 2) ; dans laquelle une combinaison desdites étroites bandes conductrices et de ladite couche isolante (4) configurée crée une ou une pluralité de parties conductrices désisolées (5) dans chacune desdites étroites bandes conductrices en tant que bornes et une partie conductrice désisolée (5) dans chacune desdites étroites bandes conductrices en tant qu'électrode ayant une surface de 2,5 × 10³ µm², et un puits pour la culture de cellules sur le dessus du réseau d'électrode plane.

2. Electrode plane selon la revendication 1, dans laquelle la distance entre des électrodes adjacentes est de 10 à 1000 µm.

3. Electrode plane selon la revendication 1 ou 2, dans laquelle la couche isolante recouvrant les fils conducteurs comporte des trous sur chaque électrode et est appliquée sur sensiblement la totalité de la surface dudit substrat isolant, à l'exception du voisinage des contacts entre un circuit extérieur et les fils conducteurs.

4. Electrode plane selon la revendication 1 ou 2, dans laquelle ledit substrat isolant comprend un matériau transparent.

5. Electrode plane selon la revendication 1 ou 2, dans laquelle les électrodes et les fils conducteurs sont constitués d'un matériau transparent.

6. Electrode plane selon la revendication 1 ou 2, dans laquelle les électrodes et les fils conducteurs sont constitués d'oxyde d'indium-étain, d'oxyde d'étain, de Cr, Au, Cu, Ni ou Al.

7. Electrode plane selon la revendication 1 ou 2, dans laquelle ladite couche isolante comprend un matériau transparent.

8. Electrode plane selon la revendication 1 ou 2, dans laquelle ladite couche isolante est constituée de résine polyimide, de résine polyimide photosensible négative, de résine époxy, de résine acrylate, de résine polyester ou de résine polyamide.

9. Electrode plane selon la revendication 1 ou 2, dans laquelle la résistance de surface de ladite électrode est égale ou inférieure à 10 Ω/cm².

10. Electrode plane selon la revendication 1 ou 2, dans laquelle les surfaces desdites électrodes sont recouvertes de platine, de noir de platine ou d'or.

11. Electrode plane selon la revendication 1 ou 2, dans laquelle les centres des électrodes sont placés à chaque intersection d'un réseau 8 × 8.

12. Electrode plane selon la revendication 3, dans laquelle un ou plusieurs éléments parmi le substrat isolant, les électrodes, les fils conducteurs et la couche isolante sont constitués d'un matériau transparent.

13. Electrode plane selon la revendication 12, dans laquelle un ou les deux éléments parmi les électrodes et les fils conducteurs sont constitués d'oxyde d'indium-étain, d'oxyde d'étain, de Cr, Au, Cu, Ni ou Al.

14. Electrode plane selon la revendication 12, dans laquelle la couche isolante est constituée de résine polyimide, de résine polyimide photosensible négative, de résine époxy, de résine acrylate, de résine polyester ou de résine polyamide.

15. Electrode plane selon la revendication 3, dans laquelle les surfaces desdites électrodes sont recouvertes de platine, de noir de platine ou d'or.

16. Electrode plane selon la revendication 3, dans laquelle les centres des électrodes sont placés à chaque intersection d'un réseau 8 × 8.

17. Utilisation d'un réseau d'électrode plane selon l'une quelconque des revendications précédentes pour capturer et/ou générer une forme d'onde de signal d'activité électrique dans un tissu et des cellules biologiques.

18. Utilisation selon la revendication 17, dans laquelle lesdits tissu et cellules biologiques sont prélevés à partir d'un système nerveux.

19. Utilisation selon les revendications 17 ou 18, dans laquelle l'activité électrique avant et après une longue stimulation, de préférence de plus d'une semaine, est comparée.

20. Utilisation selon l'une quelconque des revendications 17 à 19, dans laquelle un courant de stimulation est appliqué à un tissu ou à des cellules biologiques à une certaine électrode et dans laquelle les activités électriques du tissu ou des cellules biologiques correspondant au courant de stimulation sont enregistrées aux autres électrodes.
